# EUROPEAN PATENT APPLICATION

(11) **EP 2 452 695 A2**
(43) Date of publication of application: **16.05.2012**
(21) Application number: 11189412.7
(22) Date of filing: 23.12.2005
(51) Int. Cl.: A61K 51/04, C07B 59/00

(54) **Positron emission tomography imaging method**

(30) Priority: 23.12.2004 US 638924 P
(62) Divisional of application: 05855293.6
(71) Applicant: Purdue Research Foundation, West Lafayette, IN 47906 (US); Endocyte, Inc., West Lafayette, IN 47906 (US)
(72) Inventor: Low, Philip, West Lafayette, Indiana 47906 (US); Varghese, Bindu, West Lafayette, Indiana 47906 (US); Vlahov, Iontcho, Radoslavov, West Lafayette, Indiana 47906 (US)
(74) Representative: Elsy, David

(57) **Abstract**

The invention provides A compound of the formula

L-X

wherein L is selected from folate, folinic acid, pteropolyglutamic acid, a tetrahydropterin, a dihydrofolate, a tetrahydrofolate, a 1-deaza folate, a 3-deaza folate, a 5-deaza folate, an 8-deaza folate, a 10-deaza folate, a 1,5 dideaza folate, a 5,10-dideaza folate, an 8,10-dideaza folate, a 5,8-dideaza folate, aminopterin, amethopterin, N¹⁰ -methylfolate, 2-deamino-hydroxyfolate, 1-deazamethopterin, 3-deazamethopterin, and 3'5'-dichloro-4-amino-4-deoxy-N¹⁰ -methylpteroylglutamic acid,
wherein X comprises a radiophore comprising a radioisotope;
wherein the radiophore comprises a group selected from a benzamidyl, a benzylic, a phenyl, a naphthyl, and a benzoxazolyl group; and
wherein the radioisotope is ¹⁸F.

## Description

### CROSS REFERENCE TO RELATED APPLICATIONS

This application claims priority under 35 U.S.C. § 119(e) to U.S. Provisional Application Serial Number 60/638,924, filed on December 23, 2004, incorporated herein by reference.

### FIELD OF THE INVENTION

This invention relates to compositions and methods to diagnose/monitor, using positron emission tomography, pathogenic disease states wherein the pathogenic cells uniquely express, preferentially express, or overexpress vitamin receptors. More particularly, vitamins, or analogs thereof, conjugated to a radiophore useful in positron emission tomography are used to diagnose and monitor such disease states using an extra-corporeal device.

### BACKGROUND

Vitamin receptors are overexpressed on cancer cells. For example, the folate receptor, a 3 8 KD GPI-anchored protein that binds the vitamin folic acid with high affinity (< 1 nM), is overexpressed on ovarian, breast, bronchial, and brain cancers. Following receptor binding, rapid endocytosis delivers folate into the cell, where it is unloaded in an endosomal compartment at low pH. Importantly, covalent conjugation of small molecules, proteins, and even liposomes to folate does not block the ability of the folate to bind to its receptor, and therefore, folate conjugates can readily be delivered to and can enter cells by receptor-mediated endocytosis.

Because most cells use an unrelated reduced folate carrier to acquire the necessary folic acid, expression of the folate receptor is restricted to a few cell types. With the exception of kidney, choroid plexus, and placenta, normal tissues express low or nondetectable levels of the folate receptor. However, many malignant tissues, including ovarian, breast, bronchial, and brain cancers express significantly elevated levels of the receptor. In fact, it is estimated that 95% of all ovarian carcinomas overexpress the folate receptor.

It has also been reported that the folate receptor β, the nonepithelial isoform of the folate receptor, is expressed on activated (but not resting) synovial macrophages. Thus, folate receptors are expressed on a subset of macrophages (i.e., activated macrophages). Activated macrophages can participate in the immune response by nonspecifically engulfing and killing foreign pathogens within the macrophage, by displaying degraded peptides from foreign proteins on the macrophage cell surface where they can be recognized by other immune cells, and by secreting cytokines and other factors that modulate the function of T and B lymphocytes, resulting in further stimulation of immune responses. Activated macrophages can also contribute to the pathophysiology of disease in some instances. For example, activated macrophages can contribute to atherosclerosis, rheumatoid arthritis, autoimmune disease states, and graft versus host disease, among other disease states. It has also been shown that activated monocytes overexpress the folate receptor. The overexpression of folate receptors on activated macrophages, and on activated monocytes, is described in U.S. Patent Application Nos. 60/696,740 and U.S. Patent Application Publication No. US-2002-0192157-A1 incorporated herein by reference.

An example of the contribution of activated macrophages to disease states is the involvement of activated macrophages in the progression of atherosclerosis. Atherosclerosis is a disease state initiated when a fatty streak forms within a blood vessel wall. Formation of fatty streaks is believed to result from accumulation of lipoprotein particles in the intima layer of the blood vessel wall, the layer of the vessel wall underlying the luminal endothelial cell layer. Lipoprotein particles can associate with extracellular matrix components in the intima layer and can become inaccessible to plasma antioxidants, resulting in oxidative modification of the lipoprotein particles. Such oxidative modification may trigger a local inflammatory response resulting in adhesion of activated macrophages and T lymphocytes to the luminal endothelium followed by migration into the intima layer. The oxidized lipoprotein particles themselves can act as chemoattractants for cells of the immune system, such as macrophages and T cells, or can induce cells in the vascular wall to produce chemoattractants. The atherosclerotic lesion may then form a fibrous cap with a lipid-rich core filled with activated macrophages. Atherosclerotic lesions that are unstable are characterized by local inflammation, and lesions that have ruptured and have caused fatal myocardial infarction are characterized by an infiltration of activated macrophages and T lymphocytes.

U.S. Patent No. 6,782,289, U.S. Patent Application Publication No. US-2005-0244336-A1, and PCT International Publication No. W02004/110250 provide discussions of possible origins of blood vessel disease. The references disclose catheter-based systems for detection of radiolabeled conjugates that bind to activated macrophages within a blood vessel or other body lumen. U.S. Patent No. 6,782,289, U.S. Patent Application Publication No. US-2005-0244336-A1, and PCT International Publication No. W02004/110250 are incorporated herein by reference.

### SUMMARY

Thus, the invention provides compositions and methods to diagnose/monitor, using positron emission tomography, pathogenic disease states, including cancers and disease states that involve activated macrophages or activated monocytes, wherein the pathogenic cells uniquely express, preferentially express, or overexpress vitamin receptors. In one embodiment, vitamins, or analogs thereof, conjugated to a radiophore are used to diagnose and monitor such disease states extra-corporeally using positron emission tomography.

In one embodiment, a method is provided of diagnosing/monitoring a disease state mediated by activated monocytes or activated macrophages having accessible binding sites for a vitamin. The method comprises the steps of administering to a patient being evaluated for the disease state an effective amount of a conjugate of the general formula L-X, wherein L comprises a vitamin, or an analog thereof, and the group X comprises a radiophore, allowing sufficient time for the vitamin conjugate to bind to activated monocytes or activated macrophages, and diagnosing/monitoring the disease state extra-corporeally using positron emission tomography.

In another embodiment, a method is provided of diagnosing/monitoring a cancer wherein the cancer cells uniquely express, preferentially express, or overexpress vitamin receptors. The method comprises the steps of administering to a patient being evaluated for the cancer an effective amount of a conjugate of the general formula L-X, wherein L comprises a vitamin, or an analog thereof, and the group X comprises a radiophore, wherein the radiophore has a half-life of about 80 minutes to about 8 hours, allowing sufficient time for the vitamin conjugate to bind to the cancer cells, and diagnosing/monitoring the cancer extra-corporeally using positron emission tomography.

In another embodiment, a method is provided of diagnosing/monitoring active atherosclerotic plaques associated with blood vessels wherein the plaques comprise activated macrophages having accessible binding sites for a vitamin. The method comprises the steps of administering to a patient being evaluated for atherosclerosis an effective amount of a conjugate of the general formula L-X, wherein L comprises a vitamin, or an analog thereof, and the group X comprises a radiophore capable of decaying by emission of positrons, allowing sufficient time for the vitamin conjugate to bind to activated macrophages associated with active plaques, and diagnosing/monitoring the active plaques extra-corporeally using positron emission tomography.

Many unstable (i.e., active) atherosclerotic plaques are capable of rupturing and causing acute atherosclerotic syndromes, but do not produce luminal narrowing of blood vessels, particularly in the coronary circulation. Thus, this embodiment represents a significant advance in diagnosing the risk of myocardial infarction, and in evaluating the need for clinical intervention, in patients suffering from atherosclerosis.

In another embodiment, a composition is provided comprising a vitamin, or an analog or derivative thereof, and a positron-emitting isotope, wherein said isotope emits a pair of annihilation photons moving in opposite directions, wherein the annihilation photons are produced as a result of positron annihilation with an electron, and wherein said isotope has a half-life of from about 80 minutes to about 8 hours.

In yet another illustrative embodiment, a compound is provided of the formula L-X wherein L comprises a vitamin, or an analog or derivative thereof, and wherein X comprises a radiophore comprising a radioisotope that decays with a half-life of from about 80 minutes to about 8 hours by emission of positrons, wherein said radioisotope emits a pair of annihilation photons moving in opposite directions, and wherein the annihilation photons are produced as a result of positron annihilation with an electron.

In still another illustrative embodiment a method is provided for preparing a conjugate of the general formula L-X wherein L comprises a vitamin, or an analog or derivative thereof, and the group X comprises a radiophore comprising a radioisotope that decays by emission of positrons, wherein said radioisotope emits a pair of annihilation photons moving in opposite directions, wherein the annihilation photons are produced as a result of positron annihilation with an electron, and wherein said radioisotope has a half-life of from about 80 minutes to about 8 hours, the method comprising the steps of providing the vitamin, or analog or derivative thereof, in a reactive form capable of reacting with a radiophore in reactive form, providing the radiophore in the reactive form capable of reacting with the vitamin in reactive form, and contacting the reactive form of the vitamin with the reactive form of the radiophore.

### BRIEF DESCRIPTION OF THE DRAWINGS

Fig. 1 shows the HPLC chromatogram of crude folate-succinimidyl-fluorobenzoate (folate-SFB).
Fig. 2 shows the mass spectrum of crude folate-SFB.
Fig. 3 shows the mass spectrum of purified folate-SFB.
Fig. 4 shows the results of a competitive binding assay where radioactivity bound to KB cells was measured for KB cells alone (first bar), KB cells incubated with ³H-folate (second bar), KB cells incubated with ³H-folate in the presence of 100 nM folate-SFB (third bar), or KB cells incubated with ³H-folate in the presence of 10 µM folate-SFB (fourth bar).
Fig. 5 shows the HPLC chromatogram of folate-fluorobenzaldehyde (folate-FBA).
Fig. 6 shows the mass spectrum of folate-FBA

### DETAILED DESCRIPTION OF THE INVENTION

The present invention utilizes a compound that emits radiation (i.e., a radiophore) and which is useful in a diagnostic/monitoring method employing positron emission tomography (i.e., a compound that emits positron radiation capable of producing a pair of annihilation photons moving in opposite directions, the annihilation photons being produced as a result of positron annihilation with an electron). The radiophore typically comprises a radioisotope linked to another chemical structure (*e*.*g*., a benzene ring) to form the compound that emits radiation (i.e., the radiophore). However, the radiophore can comprise the radioisotope alone.

In accordance with the invention a compound (*e.g.*, a radiophore) "useful in positron emission tomography" means a compound that emits positron radiation capable of producing a pair of annihilation photons moving in opposite directions, the annihilation photons being produced as a result of positron annihilation with an electron.

Also, in accordance with the invention, the use of positron emission tomography (PET) requires that the compound (*e*.*g.*, the radiophore) used in PET emit positron radiation capable of producing a pair of annihilation photons moving in opposite directions, the annihilation photons being produced as a result of positron annihilation with an electron.

The vitamins, or analogs thereof, or other ligands conjugated to a radiophore useful in PET, are used to diagnose and/or monitor disease states using an extra-corporeal device. PET detection using an extra-corporeal device is also referred to as a "PET scan," and devices for extra-corporeal detection using PET are well known in the art.

The present invention relates to compositions and methods to diagnose/monitor, using PET, pathogenic disease states wherein the pathogenic cells uniquely express, preferentially express, or overexpress vitamin receptors or other receptors. The invention is applicable to populations of pathogenic cells that cause a variety of pathologies such as cancer. Thus, the population of pathogenic cells may be a cancer cell population that is tumorigenic, including benign tumors and malignant tumors, or it can be non-tumorigenic. The cancer cell population may arise spontaneously or by such processes as mutations present in the germline of the patient or somatic mutations, or it may be chemically-, virally-, or radiation-induced. The invention can be utilized to diagnose/monitor such cancers as carcinomas, sarcomas, lymphomas, Hodgekin's disease, melanomas, mesotheliomas, Burkitt's lymphoma, nasopharyngeal carcinomas, leukemias, and myelomas. The cancer cell population can include, but is not limited to, oral, thyroid, endocrine, skin, gastric, esophageal, laryngeal, pancreatic, colon, bladder, bone, ovarian, cervical, uterine, breast, testicular, prostate, rectal, kidney, liver, and lung cancers.

The pathogenic cells can also be activated monocytes or macrophages associated with disease states such as fibromyalgia, rheumatoid arthritis, osteoarthritis, ulcerative colitis, Crohn's disease, psoriasis, osteomyelitis, multiple sclerosis, atherosclerosis, pulmonary fibrosis, sarcoidosis, systemic sclerosis, organ transplant rejection (GVHD), lupus erythematosus, Sjögren's syndrome, glomerulonephritis, inflammations of the skin (e.g., psoriasis), chronic inflammations, and inflammations due to injury (e.g., a head or spinal cord injury or an embolism).

In one embodiment, a method is provided of diagnosing/monitoring a disease state mediated by activated macrophages or activated monocytes having accessible binding sites for a vitamin. The method comprises the steps of administering to a patient being evaluated for the disease state an effective amount of a conjugate of the general formula L-X, wherein L comprises a vitamin, or an analog thereof, and the group X comprises a radiophore, allowing sufficient time for the vitamin conjugate to bind to activated monocytes or the activated macrophages, and diagnosing/monitoring the disease state extra-corporeally using positron emission tomography.

In another embodiment, a method is provided of diagnosing/monitoring a cancer wherein the cancer cells uniquely express, preferentially express, or overexpress vitamin receptors. The method comprises the steps of administering to a patient being evaluated for the cancer an effective amount of a conjugate of the general formula L-X, wherein L comprises a vitamin, or an analog thereof, and the group X comprises a radiophore, wherein the radiophore has a half-life of about 80 minutes to about 8 hours, allowing sufficient time for the vitamin conjugate to bind to the cancer cells, and diagnosing/monitoring the cancer extra-corporeally using positron emission tomography.

In another embodiment of the invention, a method is provided of diagnosing/monitoring active atherosclerotic plaques associated with blood vessels wherein the plaques comprise activated macrophages having accessible binding sites for a vitamin. The method comprises the steps of administering to a patient being evaluated for atherosclerosis an effective amount of a conjugate of the general formula L-X, wherein L comprises a vitamin, or an analog thereof, and the group X comprises a radiophore capable of decaying by emission of positrons, allowing sufficient time for the vitamin conjugate to bind to the activated macrophages associated with active plaques, and diagnosing/monitoring the active plaques extra-corporeally using positron emission tomography.

This embodiment relates to a method of diagnosing/monitoring active atherosclerotic plaques in blood vessel walls. A ligand (e.g., a vitamin, or an analog thereof) that binds to a receptor which is preferentially expressed, uniquely expressed, or overexpressed on the surface of activated macrophages relative to resting macrophages, is conjugated to a radiophore. The conjugates are administered to a patient being evaluated for atherosclerosis. The conjugates bind to activated macrophages associated with active atherosclerotic plaques. The radiation emitted by the radiophore is detected extra-corporeally using positron emission tomography. Accordingly, the conjugates can be used to distinguish active atherosclerotic plaques, containing activated macrophages, from inactive plaques wherein the plaques are present in the arteries or veins of a patient being evaluated for atherosclerosis.

Many unstable (i.e., active) atherosclerotic plaques are capable of rupturing and causing acute atherosclerotic syndromes, but do not produce luminal narrowing of blood vessels, particularly in the coronary circulation. Thus, the method of the present invention represents a significant advance in diagnosing the risk of myocardial infarction, and in evaluating the need for clinical intervention, in patients suffering from atherosclerosis.

In accordance with embodiments where the conjugates bind to activated monocytes or macrophages, the conjugates can be formed from a wide variety of ligands and radiophores, including any ligand that binds to a receptor overexpressed, uniquely expressed, or preferentially expressed on the surface of activated monocytes or activated macrophages that is not expressed/presented or is not present in significant amounts on the surface of resting monocytes or macrophages. For activated macrophages, such ligands include N-formyl peptides (e.g., f-Met-Leu-Phe), high mobility group factor 1 protein (HMGB1), hyaluronan fragments, HSP-70, toll-like receptor ligands, scavenger receptor ligands, co-receptors for antigen presentation, ligands that bind to the CD68, BER-MAC3, RFD7, CD4, CD 14, and HLA-D markers on activated macrophages, ligands that bind to urokinase plasminogen activator receptors (e.g., the WX-360 peptide), antibodies, or fragments thereof, that bind preferentially to activated macrophages, and vitamins or receptor-binding vitamin analogs/derivatives.

For monocytes, the monocyte-binding ligand can be any ligand that binds to a receptor expressed or overexpressed on activated monocytes including CD40-, CD16-, CD 14-, CD11b-, and CD62-binding ligands, 5-hydroxytryptamine, macropahge inflammatory protein 1-α, MIP-2, receptor activator of nuclear factor kB ligand antagonists, monocyte chemotactic protein 1-binding ligands, chemokine receptor 5-binding ligands, RANTES-binding ligands, chemokine receptor-binding ligands, and vitamins or receptor-binding vitamin analogs/derivatives, and the like. The conjugates are capable of preferentially binding to activated monocytes or activated macrophages compared to resting monocytes or macrophages due to preferential expression of the receptor for the ligand on activated monocytes or macrophages.

In the above-described embodiments, the ligand (e.g., a vitamin or an analog or derivative thereof) can be any ligand that binds to a receptor which is preferentially expressed, uniquely expressed, or overexpressed the surface of cancer cells, or activated monocytes or activated macrophages relative to resting monocytes or macrophages. Exemplary of such ligands are vitamins selected from the group consisting of folate receptor-binding ligands, biotin receptor-binding ligands, vitamin B₁₂ receptor-binding ligands, riboflavin receptor-binding ligands, thiamine receptor-binding ligands, and other vitamin receptor-binding ligands, or analogs or derivatives thereof.

Acceptable vitamin moieties that can be used in accordance with the invention include niacin, pantothenic acid, folic acid, riboflavin, thiamine, biotin, vitamin B₁₂, and the lipid soluble vitamins A, D, E and K. In one embodiment, these vitamins, and their receptor-binding analogs and derivatives, constitute the targeting entity that can be coupled with a radiophore, capable of emitting radiation, to form the conjugates for use in accordance with the invention. Preferred vitamin moieties include folic acid, biotin, riboflavin, thiamine, vitamin B₁₂, and receptor-binding analogs and derivatives of these vitamin molecules, and other related vitamin receptor-binding molecules (see U.S. Patent No. 5,688,488, incorporated herein by reference). Exemplary of a vitamin analog is a folate analog containing a glutamic acid residue in the D configuration (folic acid normally contains one glutamic acid in the L configuration linked to pteroic acid).

In one embodiment, the vitamin receptor-binding ligand can be folic acid, a folic acid analog, or another folate receptor-binding molecule. Analogs of folate that can be used include folinic acid, pteropolyglutamic acid, and folate receptor-binding pteridines such as tetrahydropterins, dihydrofolates, tetrahydrofolates, and their deaza and dideaza analogs. The terms "deaza" and "dideaza" analogs refers to the art recognized analogs having a carbon atom substituted for one or two nitrogen atoms in the naturally occurring folic acid structure. For example, the deaza analogs include the 1-deaza, 3-deaza, 5-deaza, 8-deaza, and 10-deaza analogs. The dideaza analogs include, for example, 1,5 dideaza, 5,10-dideaza, 8,10-dideaza, and 5,8-dideaza analogs. The foregoing folic acid analogs are conventionally termed "folates," reflecting their capacity to bind to folate receptors. Other folate receptor-binding analogs include aminopterin, amethopterin (methotrexate), N¹⁰-methylfolate, 2-deamino-hydroxyfolate, deaza analogs such as 1-deazamethopterin or 3-deazamethopterin, and 3',5'-dichloro-4-amino-4-deoxy-N¹⁰-methylpteroylglutamic acid (dichloromethotrexate).

In all of the above-described embodiments, the radiophore may include a positron-emitting isotope having a suitable half-life and toxicity profile. In various embodiments, the radioisotope has a half-life of more than 30 minutes, more than 70 minutes, more than 80 minutes, more than 90 minutes, more than 100 minutes, less than 8 hours, less than 6 hours, less than 4 hours, or less than 3 hours. In other embodiments, the radioisotope has a half-life of about 30 minutes to about 4 hours, about 70 minutes to about 4 hours, about 80 minutes to about 4 hours, about 90 minutes to about 4 hours, about 100 minutes to about 4 hours, about 30 minutes to about 6 hours, about 70 minutes to about 6 hours, about 80 minutes to about 6 hours, about 90 minutes to about 6 hours, about 100 minutes to about 6 hours, about 30 minutes to about 8 hours, about 70 minutes to about 8 hours, about 80 minutes to about 8 hours, about 90 minutes to about 8 hours, or about 100 minutes to about 8 hours.

In various embodiments, the radioisotope is selected from group consisting of ³⁴Cl, ⁴⁵Ti, ⁵¹Mn, ⁶¹Cu, ⁶³Zn, ⁶⁸Ga, ¹¹C, ¹³N, ¹⁵O, and ¹⁸F. In one illustrative embodiment, the radioisotope is ¹⁸F.

In the conjugates of the general formula L-X in accordance with the present invention, the group L is a ligand capable of binding to cancer cells or activated monocytes or activated macrophages as compared to resting monocytes or macrophages as described above. In one illustrative embodiment, the cancer cell or activated monocyte or activated macrophage binding ligand is folic acid, a folic acid analog/derivative, or other folate receptor-binding molecule.

The binding site for the ligand (e.g., a vitamin or an analog or derivative thereof) can include receptors for any ligand molecule capable of preferentially binding to a receptor uniquely expressed, overexpressed, or preferentially expressed/presented on the surface of cancer cells or activated monocytes or activated macrophages. A surface-presented protein uniquely expressed, overexpressed, or preferentially expressed by cancer cells or activated monocytes or activated macrophages is a receptor that is either not present or is present at insignificant concentrations on normal cells or on resting monocytes or resting macrophages providing a means for preferential detection of cancer cells or activated monocytes or activated macrophages. Accordingly, any receptor that is upregulated on cancer cells or activated monocytes or activated macrophages compared to resting monocytes or resting macrophages, or which is not expressed/presented on the surface of normal cells or resting monocytes or resting macrophages, or any receptor that is not expressed/presented on the surface of cancer cells or resting monocytes or resting macrophages in significant amounts could be used for targeting. In one illustrative embodiment, the site that binds the conjugates used in accordance with the present invention is a vitamin receptor, for example, the folate receptor, which binds folate or an analog or derivative thereof.

In accordance with the invention the conjugates can bind with high affinity to receptors on cancer cells or activated monocytes or activated macrophages. The high affinity binding can be inherent to the ligand or the binding affinity can be enhanced by the use of a chemically modified ligand (i.e., an analog or a derivative) or by the particular chemical linkage, in the conjugate, between the ligand and the radiophore.

The chemical linkage in the conjugate between the ligand and the radiophore can be a direct linkage or can be through an intermediary linker. If present, an intermediary linker can be any biocompatible linker known in the art. Typically, the linker comprises about 1 to about 30 carbon atoms, more typically about 2 to about 20 carbon atoms. Lower molecular weight linkers (i.e., those having an approximate molecular weight of about 30 to about 500) are typically employed. Any linkers or linking methods or chemistry described in U.S. Patent Application Serial Nos. 10/765336 or 60/590580, incorporated herein by reference, can be used. Any linkers or linking methods or chemistry known in the art can also be used.

Generally, any manner of forming a conjugate between the ligand and the radiophore, between a linker and the ligand, or between a linker and the radiophore can be utilized in accordance with the present invention. With or without a linker, the conjugate can be formed by conjugation of the components of the conjugate, for example, through hydrogen, ionic, or covalent bonds. Covalent bonding of the components of the conjugate can occur, for example, through the formation of amide, ester, disulfide, or imino bonds between acid, aldehyde, hydroxy, amino, sulfhydryl, or hydrazo groups. Also, in accordance with this invention a linker can comprise an indirect means for associating the ligand with the radiophore, such as by connection through spacer arms or bridging molecules. Both direct and indirect means for association should not prevent the binding of the ligand to the receptor on the cancer cells or activated monocytes or activated macrophages for operation of the method of the present invention.

As an illustrative chemical linkage, ethylene diamine in the scheme described in Example 1 serves as a spacer or linker between the fluorobenzamide (i.e., the radiophore where the fluorine molecule is ¹⁸F) and the folate ligand. Preferably the linker contributes to water solubility of the conjugate, or at least does not materially detract from water solubility. Advantageous linkers for water solubility include water soluble polymers such as dextran, cellulose ethers, peptide linkers, or polyethylene glycol. In one embodiment, such polymers have a molecular weight of less than 1,000.

In illustrative embodiments, in addition to radiophores comprising benzamidyl, benzylic, or phenyl groups, other aromatic groups, such as, for example, naphthyl and benzoxazolyl groups, and the like are contemplated to be within the scope of the invention.

By appropriate selection, linkers may limit the rate of excretion of the conjugate from the patient by permitting the ligand to associate with the site of interest, such as cancer cells or activated monocytes or activated macrophages before being excreted in the bile from the liver, or in the urine. A linker may facilitate, or may delay metabolic consumption of the conjugate such as by retarding reticuloendothelial system uptake, particularly by the liver. A linker may also help avoid association of the conjugate with non-target organs, cells, fluids, or proteins. If, for example, the conjugate associated with a serum protein, the PET scan would provide a scan of the patient's blood vessels generally, in contrast to the specific location of cancer cells or activated monocytes or activated macrophages sought. Also, the linker may facilitate or accelerate a preferred route of excretion of the conjugate, such as through urine, for example, by encouraging the patient to drink significant fluids after the administration of the conjugate.

In the embodiment where the ligand is folic acid, an analog/derivative of folic acid, or any other folate receptor-binding molecule, the folate, or analog/derivative thereof, can be conjugated to the linker by an art-recognized procedure that utilizes trifluoroacetic anhydride to prepare γ-esters of folic acid via a pteroyl azide intermediate. This procedure results in the synthesis of folate, conjugated to the linker only through the γ-carboxy group of the glutamic acid groups of folate. Alternatively, folic acid analogs can be coupled by art-recognized procedures through the α-carboxy moiety of the glutamic acid group or both the α and γ carboxylic acid entities.

The amount of the conjugate effective for use in accordance with the method of the invention depends on many parameters, including the molecular weight of the conjugate, its route of administration, and its tissue distribution. In accordance with the invention an "effective amount" of the conjugate is an amount sufficient to bind to cancer cells or activated monocytes or activated macrophages and to be useful in the diagnosis/monitoring of cancer or disease states involving activated monocytes or activated macrophages. The effective amount of the conjugate to be administered to a patient being evaluated for cancer or disease states involving activated monocytes or activated macrophages can range from about 1 pg/kg to about 10 mg/kg, 1 ng/kg to about 10 mg/kg, or from about 10 µg/kg to about 1 mg/kg, or from about 100 µg/kg to about 500 µg/kg.

The conjugate can be administered in one or more doses (*e.g.*, about 1 to about 3 doses) prior to detection with the extra-corporeal PET imaging device. The number of doses depends on the molecular weight of the conjugate, its route of administration, and its tissue distribution, among other factors. When used for diagnosis/monitoring of cancer or disease states involving activated monocytes or activated macrophages, the extra-corporeal detection procedure is typically performed about 1 minute to about 6 hours post-administration of the conjugate, but the extra-corporeal detection procedure can be performed at any time post-administration of the conjugate as long as binding of the conjugate to cancer cells or activated monocytes or activated macrophages is detectable and sufficient time is allowed for elimination of a substantial fraction of the unbound conjugate from the body.

The conjugates administered in accordance with the method of this invention are preferably administered parenterally to the patient, for example, intravenously, intradermally, subcutaneously, intramuscularly, or intraperitoneally, in combination with a pharmaceutically acceptable carrier. Alternatively, the conjugates can be administered to the patient by other medically useful procedures such as in an orally available formulation. In accordance with the invention, any patient suspected of having cancer or a disease state involving activated monocytes or activated macrophages, whether symptomatic or not, who would benefit from an evaluation using the method of the present invention can be evaluated.

The conjugates used in accordance with this invention of the formula L-X are used in one aspect of this invention to formulate diagnostic compositions comprising effective amounts of the conjugate and an acceptable carrier therefor. Examples of parenteral dosage forms include aqueous solutions of the conjugate, for example, a solution in isotonic saline, 5% glucose or other well-known pharmaceutically acceptable liquid carriers such as alcohols, glycols, esters and amides. Any orally available dosage forms known in the art can also be used.

The conjugates use in the methods described herein are formed to target and, thus, to concentrate the conjugate at the site of a tumor or at the site of accumulation of activated monocytes or activated macrophages (*e.g.*, activated macrophages adhering to the luminal endothelial layer of the plaque or activated macrophages present in the lipid-rich core of the plaque) in the patient.

Several aspects of the present invention are advantageous in the detection of cancer cells or activated monocytes or activated macrophages. In one embodiment, the radiophore comprises an elemental isotope which is a positron emitter. Positron emitters emit in three dimensions from the source atom, but the emission proceeds in two parts in exactly opposite directions. As the anti-particle of the electron, when the positron from a decaying isotope comes in contact with electrons in nearby matter, it annihilates emitting energy from the annihilation as gamma rays. To conserve momentum, the gamma ray photons travel in opposite directions. Because the positron has two radiation rays available for detection, the location in the patient where the conjugate has accumulated is more readily and therefore more accurately, detected within a time frame reasonable for patient diagnosis. The signal-to-noise ratio of positron annihilation is markedly improved over unidirectional gamma rays. Further, by back-projecting coincident rays, the location of the source emission is located.

PET is presently used in medical centers as a diagnostic tool for the detection of cancer. In cancer diagnosis, a patient may be administered glucose tagged with a positron emitter (e.g., fluorodeoxyglucose labeled with ¹⁸F). Glucose concentrates in fast-growing cancer cells. The presence of a cancer may be detected by the concentration of the PET imaging agent. Also, the location of the cancer in the body is determined by back-projecting the coincident gamma radiation by means of the PET scanner. Thus, the method of the present invention may be used in combination with fluorodeoxyglucose labeled with ¹⁸F to detect cancer cells. The method of the invention may also be used in combination with any other methods of cancer diagnosis already developed and known in the art, including methods using other already developed diagnostic agents and utilizing x-ray computed tomography (CT), magnetic resonance imaging (MRI), functional magnetic resonance imaging (fMRI), ultrasound, and single photon emission computed tomography (SPECT).

In other embodiments, the method of the present invention can be used alone or in combination with any other method(s) known in the art for the detection/analysis/ablation of atherosclerotic plaques. For example, the invention can be used in combination with methods to ablate atherosclerotic plaques in cases where active plaques cause narrowing of blood vessels. In such cases, the conjugates of the present invention can be used not only to identify active atherosclerotic plaques as compared to inactive plaques, but also to distinguish between atherosclerotic and normal tissue to aid in ablation procedures. Thus, the present invention can be used to analyze both the physiological and the morphological state of atherosclerotic plaques. For example, angioplasty involves the nonsurgical widening of a vessel narrowed by plaque deposition, and laser energy, for example, directed through optical fibers in a catheter-based device, can be used to ablate or partially remove the plaque deposits. Catheter-based devices for ablating plaques using laser energy are described in U.S. Patents Nos. 4,817,601, 4,850,351, and 4,950,266, incorporated herein by reference.

The invention utilizes a useful positron emitting isotope. A suitable radiophore may be prepared using the isotope fluorine ¹⁸F. Other useful positron-emitting isotopes may also be employed. Factors deserving consideration in the selection of a suitable isotope include sufficient half-life of the positron-emitting isotope to permit preparation of a diagnostic composition in a pharmaceutically acceptable carrier prior to administration to the patent, and sufficient remaining half-life to yield sufficient activity to permit extra-corporeal measurement, for example, by a PET scan. Further, a suitable isotope should have a sufficiently short half-life to limit patient exposure to unnecessary radiation. In an illustrative embodiment, ¹⁸F, having a half-life of 110 minutes, provides adequate time for preparation of the diagnostic composition, as well as an acceptable deterioration rate.

In one illustrative embodiment, the isotope should have sufficient chemical activity to permit the isotope to become bound to a chemical compound and in turn to the ligand, whether or not a linker is used. Isotopes of elements having toxic properties should be avoided. Positron-decaying isotopes having suitable half-lives include: ³⁴Cl, half-life 32 minutes; ⁴⁵Ti, half-life 3.09 hours; ⁵¹Mn, half-life 45 minutes; ⁶¹Cu, half-life 3.41 hours; ⁶³Zn, half-life 38.4 minutes; ⁶⁸Ga, half-life 68.3 minutes, ¹¹C, half-life 20 minutes, ¹⁵O, half-life 2 minutes, ¹³N, half-life 10 minutes, or ¹⁸F, half-life 110 minutes.

The invention also provides:
1. A method of diagnosing/monitoring a disease state mediated by activated monocytes or activated macrophages having accessible binding sites for a vitamin, the method comprising the steps of:
   a. administering to a patient being evaluated for the disease state an effective amount of a conjugate of the general formula
      L-X
      wherein L comprises a vitamin, or an analog or derivative thereof, and the group X comprises a radiophore comprising a radioisotope;
   b. allowing sufficient time for the vitamin conjugate to bind to the activated monocytes or the activated macrophages; and
   c. diagnosing/monitoring the disease state extra-corporally using positron emission tomography.
2. The method of 1 wherein the vitamin is selected from the group consisting of folate, biotin, vitamin B₁₂, riboflavin, and thiamine, or vitamin receptor-binding analogs or derivatives thereof.
3. The method of 1 where the radioisotope is selected from group consisting of ³⁴Cl, ⁴⁵Ti, ⁵¹Mn, ⁶¹Cu, ⁶³Zn, ⁶⁸Ga, ¹¹C, ¹³N, ¹⁵O, and ¹⁸F.
4. The method of 3 wherein the radioisotope is ¹⁸F.
5. The method of 1 wherein the radioisotope has a half-life of about 30 minutes to about 8 hours.
6. The method of 1 wherein the radioisotope has a half-life of about 70 minutes to about 8 hours.
7. The method of 1 wherein the radioisotope has a half-life of about 80 minutes to about 8 hours.
8. The method of 1 wherein the radioisotope has a half-life of about 90 minutes to about 8 hours.
9. The method of 1 wherein the radioisotope has a half-life of about 100 minutes to about 8 hours.
10. The method of 1 wherein the conjugate includes a linker that enhances water solubility of the conjugate.
11. The method of 1 wherein the conjugate includes a linker that retards reticuloendothelial system uptake of the conjugate.
12. The method of 1 where the linker retards liver uptake of the conjugate.
13. A method of diagnosing/monitoring a cancer wherein the cancer cells uniquely express, preferentially express, or overexpress vitamin receptors, the method comprising the steps of:
   a. administering to a patient being evaluated for the cancer an effective amount of a conjugate of the general formula
      L-X
      wherein L comprises a vitamin, or an analog or derivative thereof, and the group X comprises a radiophore comprising a radioisotope wherein the radioisotope has a half-life of about 80 minutes to about 8 hours;
   b. allowing sufficient time for the vitamin conjugate to bind to the cancer cells; and
   c. diagnosing/monitoring the cancer extra-corporally using positron emission tomography.
14. The method of 13 wherein the vitamin is selected from the group consisting of folate, biotin, vitamin B₁₂, riboflavin, and thiamine, or vitamin receptor-binding analogs or derivatives thereof.
15. The method of 13 where the radioisotope in the radiophore is selected from group consisting of ⁴⁵Ti, ⁶¹Cu, and ¹⁸F.
16. The method of 15 wherein the radioisotope is ¹⁸F.
17. The method of 13 wherein the conjugate includes a linker that enhances water solubility of the conjugate.
18. The method of 13 wherein the conjugate includes a linker that retards reticuloendothelial system uptake of the conjugate.
19. The method of 13 where the linker retards liver uptake of the conjugate.
20. A method of diagnosing/monitoring active atherosclerotic plaques associated with blood vessels wherein the plaques comprise activated macrophages having accessible binding sites for a vitamin, the method comprising the steps of:
   a. administering to a patient being evaluated for atherosclerosis an effective amount of a conjugate of the general formula
      L-X
      wherein L comprises a vitamin, or an analog or derivative thereof, and the group X comprises a radiophore comprising a radioisotope capable of decaying by emission of positrons;
   b. allowing sufficient time for the vitamin conjugate to bind to activated macrophages associated with active plaques; and
   c. diagnosing/monitoring the active plaques extra-corporally using positron emission tomography.
21. The method of 20 wherein the vitamin is folate, or an analog or derivative thereof.
22. The method of 20 wherein the vitamin is selected from the group consisting of folate, biotin, vitamin B₁₂, riboflavin, and thiamine, or receptor-binding analogs or derivatives thereof.
23. The method of 20 where the radioisotope is selected from group consisting of ³⁴Cl, ⁴⁵Ti, ⁵¹Mn, ⁶¹Cu, ⁶³Zn, ⁶⁸Ga, ¹¹C, ¹³N, ¹⁵O, and ¹⁸F.
24. The method of 23 wherein the radioisotope comprises ¹⁸F.
25. The method of 20 wherein the radioisotope has a half-life of about 30 minutes to about 8 hours.
26. The method of 20 wherein the radioisotope has a half-life of about 70 minutes to about 8 hours.
27. The method of 20 wherein the radioisotope has a half-life of about 80 minutes to about 8 hours.
28. The method of 20 wherein the radioisotope has a half-life of about 90 minutes to about 8 hours.
29. The method of 20 wherein the radioisotope has a half-life of about 100 minutes to about 8 hours.
30. The method of 20 wherein the conjugate includes a linker that enhances water solubility of the conjugate.
31. The method of 20 wherein the conjugate includes a linker that retards reticuloendothelial system uptake of the conjugate.
32. The method of 20 where the linker retards liver uptake of the conjugate.
33. A composition comprising a vitamin, or an analog or derivative thereof, and a positron-emitting isotope, wherein said isotope emits a pair of annihilation photons moving in opposite directions, wherein the annihilation photons are produced as a result of positron annihilation with an electron, and wherein said isotope has a half-life of from about 80 minutes to about 8 hours.
34. The composition of 33, wherein the isotope is selected from the group consisting of ¹⁸F ⁴⁵Ti, and ⁶¹Cu.
35. The composition of 33, wherein the isotope is non-toxic.
36. The composition of 33, wherein the isotope is chemically reactive.
37. The composition of 33, wherein the vitamin is folate, or a folate receptor-binding analog or derivative thereof.
38. The composition of 37, wherein the isotope is non-toxic.
39. The composition of 33, wherein the isotope is ¹⁸F.
40. The composition of 37, wherein the isotope is ¹⁸F.
41. The composition of 33, wherein the vitamin is conjugated to the isotope through a linker.
42. The composition of 37, wherein the folate is conjugated to the isotope through a linker.
43. A compound of the formula
   L-X
   wherein L comprises a vitamin, or an analog or derivative thereof, and
   wherein X comprises a radiophore comprising a radioisotope that decays with a half-life of from about 80 minutes to about 8 hours by emission of positrons, wherein said radioisotope emits a pair of annihilation photons moving in opposite directions, and wherein the annihilation photons are produced as a result of positron annihilation with an electron.
44. The compound of 43, wherein the vitamin is selected from the group consisting of folate, biotin, vitamin B₁₂, riboflavin, and thiamine, or receptor-binding analogs or derivatives thereof.
45. The compound of 43, wherein the vitamin is folate, or an analog or derivative thereof.
46. The compound of 43, wherein the radioisotope is selected from the group consisting of ¹⁸F, ⁴⁵Ti, and ⁶¹Cu.
47. The compound of 43, wherein the vitamin is conjugated to the radiophore through a linker.
48. The compound of 47, wherein the linker is selected from the group consisting of diamines, dextrans, cellulose ethers, peptides, and polyethylene glycol.
49. The compound of 43, wherein the radiophore comprises a *para*-substituted radioisotope.
50. A method for preparing a conjugate of the general formula
   L-X
   wherein L comprises a vitamin, or an analog or derivative thereof, and the group X comprises a radiophore comprising a radioisotope that decays by emission of positrons, wherein said radioisotope emits a pair of annihilation photons moving in opposite directions, wherein the annihilation photons are produced as a result of positron annihilation with an electron, and wherein said radioisotope has a half-life of from about 80 minutes to about 8 hours, the method comprising the steps of:
   a. providing the vitamin, or analog or derivative thereof, in a reactive form capable of reacting with a radiophore in reactive form;
   b. providing the radiophore in reactive form capable of reacting with the vitamin in reactive form; and
   c. contacting the reactive form of the vitamin with the reactive form of the radiophore.
51. The method of 50, wherein the reactive form of the vitamin includes a linker.
52. The method of 51, wherein the linker is selected from group consisting of diamines, dextran, cellulose ethers, peptides, and polyethylene glycol.
53. The method of 50, wherein the reactive form of the radiophore includes an active ester.
54. The method of 50, wherein the reactive form of the radiophore includes an aldehyde.
55. The method of 50, wherein the reactive form of the radiophore includes ¹⁸F.

### EXAMPLE 1

### SYNTHESIS OF FOLATE CONJUGATE

Using a suitable isotope, a biocompatible conjugate L-X is required from which to prepare diagnostic composition. For example in the case of ¹⁸F, a suitable L-X conjugate may be prepared according to the following synthetic protocol. The conjugate synthesized according to this example has an ethylene diamine linker. 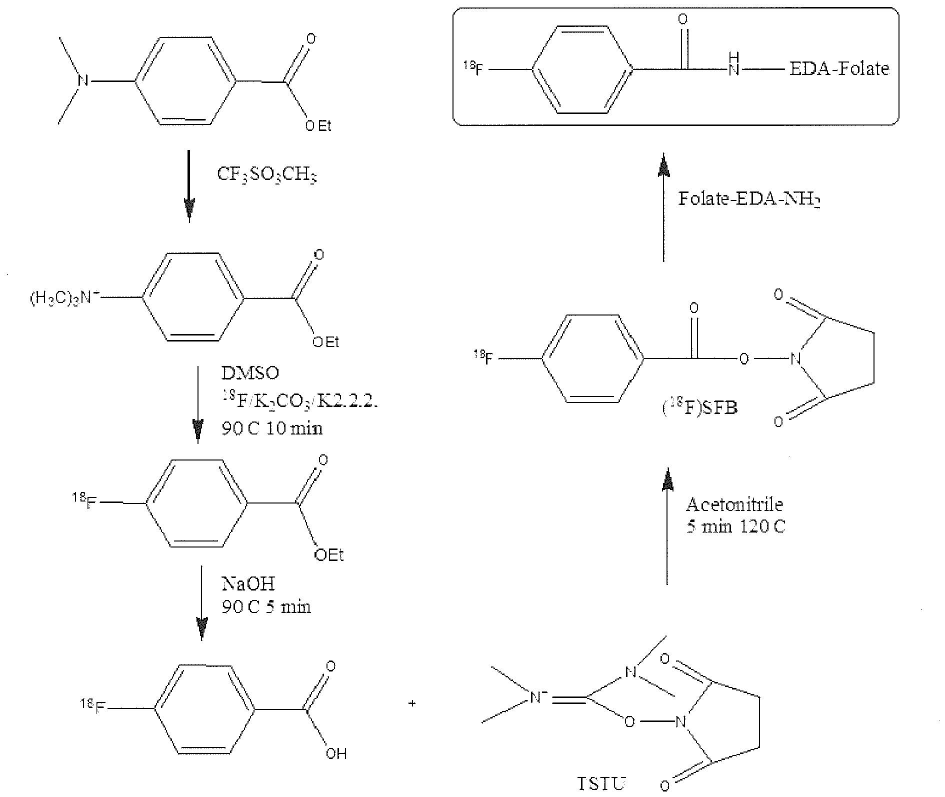 EDA = ethylene diamine
DMSO = dimethyl sufoxide
K2.2.2 = Kryptofix, Aldrich Catalog number 29,111-0
TSTU = N-Hydroxysuccinimide tetramethylurea

During the synthesis, the skilled practitioner will follow an appropriate procedure to concentrate and purify the p-fluorobenzoic acid. A typical, but by no means exclusive procedure to purify and concentrate fluorobenzoic acid, may be the addition of sufficient HCl to protinate the molecules, then isolation on a reverse phase C18 column such as a C18 SepPak Plus sold by Waters Corp. Milford Massachusetts. The column may be washed with HCl acidified water to remove any water soluble contaminants. The p-fluorobenzoic acid may be eluted from the column with methanol followed by further contaminant removal on a cationic ion-exchange column (e.g., a Dowex column), and concentration by evaporation of the methanol.

A typical, but by no means exclusive procedure to concentrate and purify the N-hydroxysuccinimide ester of p-fluorobenzoic acid may start with isolation by reverse phase high performance liquid chromatography in a mixture of water/acetonitrile/and sufficient trifluoroacetic acid to preserve an acidic pH. A water diluted solution of the ester may be concentrated on a C18 SepPak column followed by elution with diethyl ether. Residual water may be removed using a column of anhydrous Mg₂SO₄. After evaporation of the ether to dryness, the ester may be re-dissolved in acetonitrile. These procedures were used to make the above-described compound according to the described procedures.

### EXAMPLE 2

### SYNTHESIS OF FOLATE CONJUGATE

Starting from known procedures to prepare the p-fluorobenzaldehyde, a suitable conjugate may be quickly prepared as described below. Concentration and purification procedures may follow as above, or otherwise according to techniques known in the art.

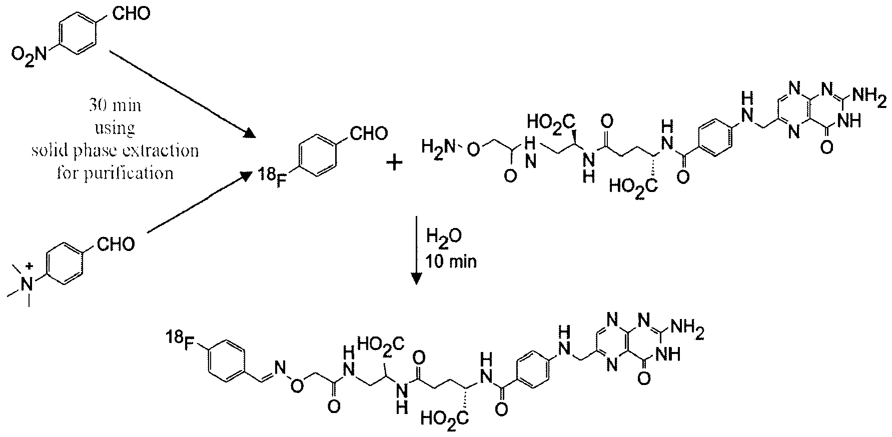

The synthesis may be completed with sufficient alacrity so as to have a significant remaining half-life of the ¹⁸F positron emitter to enable a diagnostic test.

### EXAMPLE 3

### SYNTHESIS OF FOLATE-SFB

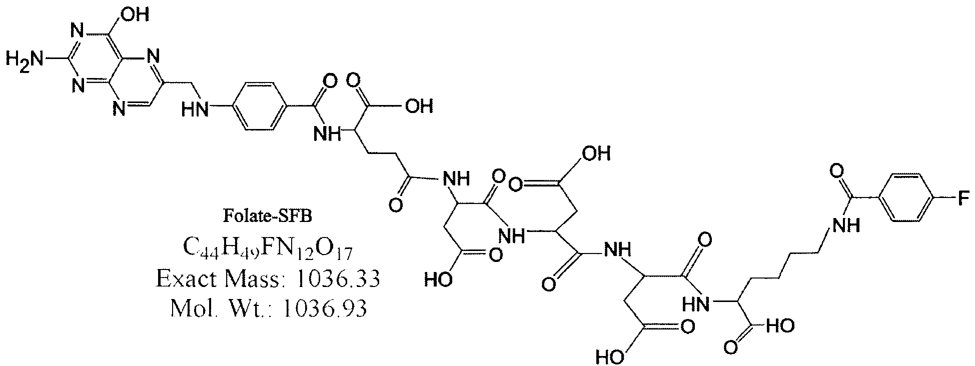

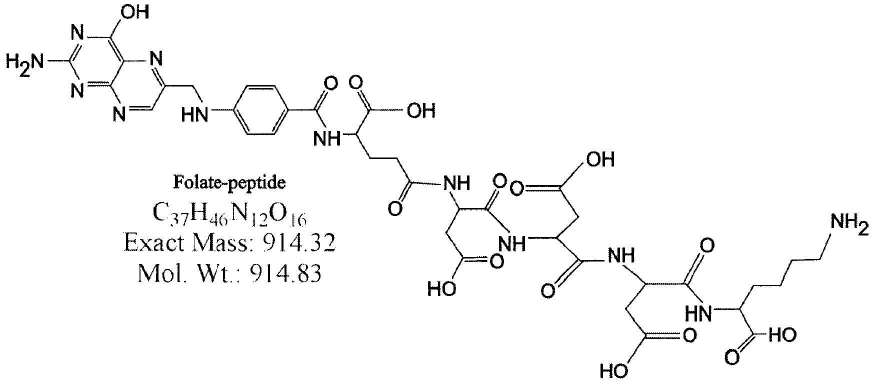

Synthesis of Folate-Peptide (Resin-Lys-Asp-Asp-Asp-Glu-Pteroic acid).

A Wang resin was attached to lysine with MTT (4-methyl-trityl) and F-moc protecting groups. The F-moc protecting groups were removed using 20% Piperdine/DMF (dimethylformamide) (10ml/g). The resin was then bubbled 10 minutes with argon and drained. The removal of the F-moc protecting groups and the bubbling steps were repeated two additional times. The resin was then washed three times with DMF. A Kaiser Test was positive for free amine groups.

Fmoc-AA (Fmoc-amino acid), HBTU (2-(1H-benzotriazole-1-yl)-1,1,3,3-tetramethyluronium hexafluorophosphate), HOBt (1-hydroxybenzotriazole), DIPEA (diisopropylethylamine) in DMF was then added and bubbled 30 minutes. Washes (three times) with DMF were then performed, and these three steps were repeated. A Kaiser Test was negative showing no free amines.

The next amino acid was then added and the above-described steps were repeated and the resin was washed with DCM (dichloromethane) for 15 minutes and bubbled in 1% TFA (trifluoroacetic acid)/DCM to remove MTT. The resin was then washed again with dichloromethane for 15 minutes and bubbled in 1% TFA/DCM. The steps were repeated until all the yellow color was gone. The resin was then washed with DCM three times and reswelled with DMF.

To Pteroic Acid (CF₃) in DMF, were addded HOBt, HBTU and DIPEA. The composition turned brown and was bubbled overnight and then washed three times with DMF. A Kaiser Test was negative. The F-moc groups were removed with 20% Piperdine/DMF (10ml/g) and the resin was bubbled 10 minutes and drained. The F-moc removal and bubbling steps were repeated two additional times. The resin was then washed three times with DMF, three times with DCM, and three times with MeOH. The resin was dried under high vacuum for 4 hours.

TFA was cleaved with a solution comprising 95:2.5:2.5 TFA:TIS(triisopropyl silane):H2O. This solution was added to the beads and bubbled 2-3 hours and drained into a clean flask. The beads were then washed with the same solution and drained until the beads were clear. The solution was Rotovaped to reduce the solvent level to 2-3 ml. Conical centrifuge tubes were then filled with 50 ml of ice-cold ether. The peptide mixture was added to the ether vial so the peptide precipitates out. The mixture was centrifuged and washed 2-3 times with ether and dried under vacuum to get rid of trace ether. The peptide was then dissolved in 1% NH₄OH to get rid of the TFA protecting group using a stir bar to mix for 30-40 minutes. The peptide was then lyophilized, purified by HPLC, and lyophilized.

### Synthesis of non-radioactive SFB (Succinimidyl-fluorobenzoate)

The synthesis of non-radioactive SFB was done by a protocol described in Eur. J. Med. Mol. Imaging, vol. 31: 469-474 (2004). Starting from fluorobenzoic acid, an oil bath was set up at 90 degrees celsius. A solution was made of 45% tetramethlyammonium hydroxide (TMAH) in water. In a separate vial, 10.0 mg of 4-fluorobenzoic acid was added (solution 1). In a separate vial, 40 ul of 45% TMAH was added (solution 2). Then 0.2 ml of water and 1.0 ml of acetonitrile was added and solution 2 was added to solution 1 (solution 3). The solution was evaporated to dryness with a rotovap. In another vial, 14 mg of TSTU was added to 1.2 ml acetonitrile (solution 4). Solution 4 was added to solution 3. The mixture was heated to 90 degrees Celsius in an oil bath for 2 minutes. Radioactive ¹⁸F will be synthesized in a cyclotron by procedures well-known in the art and used to prepare p-(¹⁸F)fluorobenzoic acid, as shown in Example 1, which will then be converted to¹⁸F-SFB as described above.

### Synthesis of Folate-SFB

Folate-peptide in PBS was combined with SFB (in acetonitrile) and the pH of folate-peptide (in PBS) was adjusted and the conjugate was examined by HPLC.

### EXAMPLE 4

### HPLC OF CRUDE FOLATE-SFB

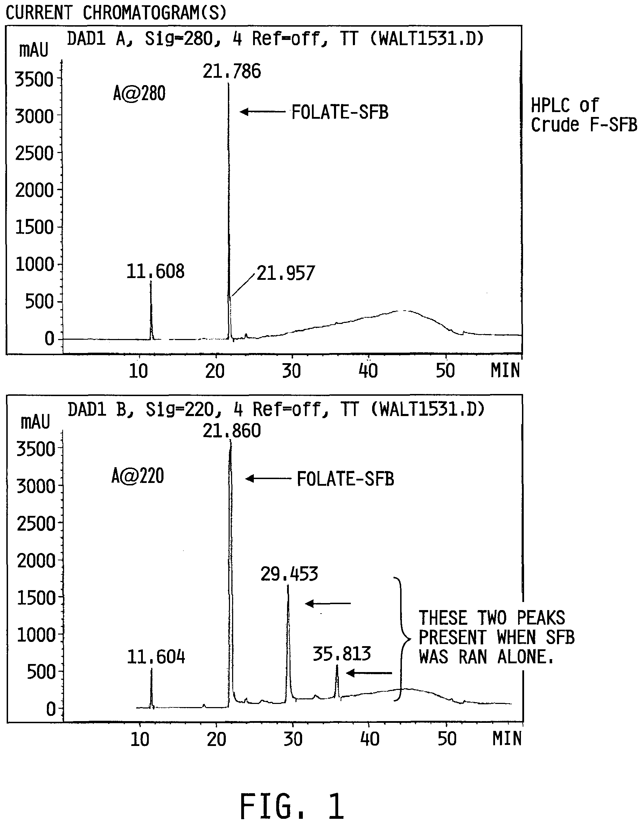

Crude folate-SFB was analyzed by high performance liquid chromatography (HPLC) using conditions similar to those described in Clinical Science, vol. 103: pp. 4S-8S (2002), but with the following modifications. The reverse phase HPLC was performed using a C18 column and the conditions were water 0.1% TFA in ACN at 77:23 for 10 min, 60:40 for 10 min, 50:50 for 10 min, 40:60 for 10 min, and using a flow rate of 1.0 ml/minute. The results are shown in Fig. 1.

### EXAMPLE 5

### MASS SPECTRA OF CRUDE AND PURIFIED FOLATE-SFB

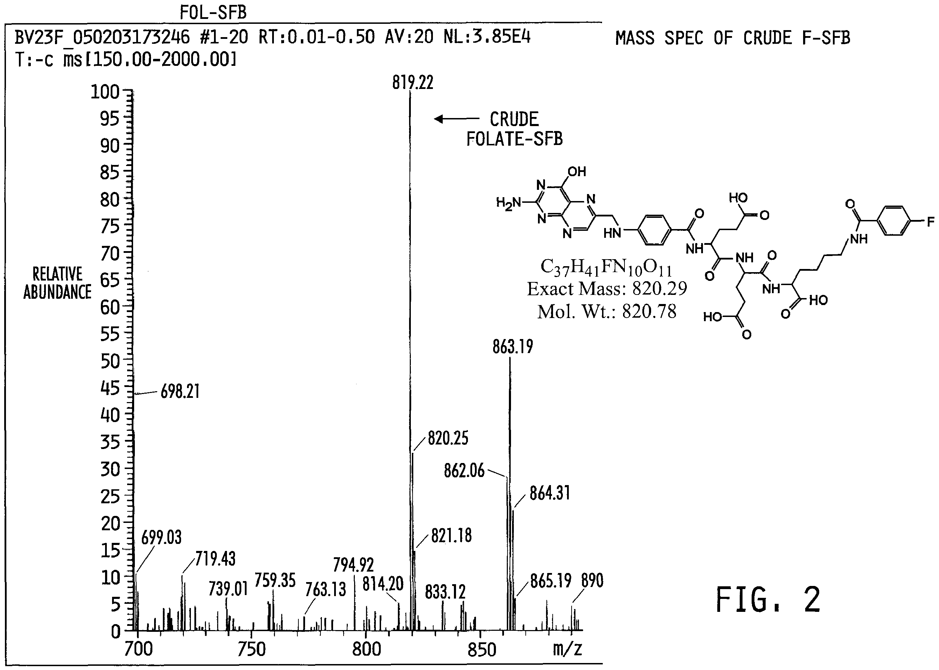

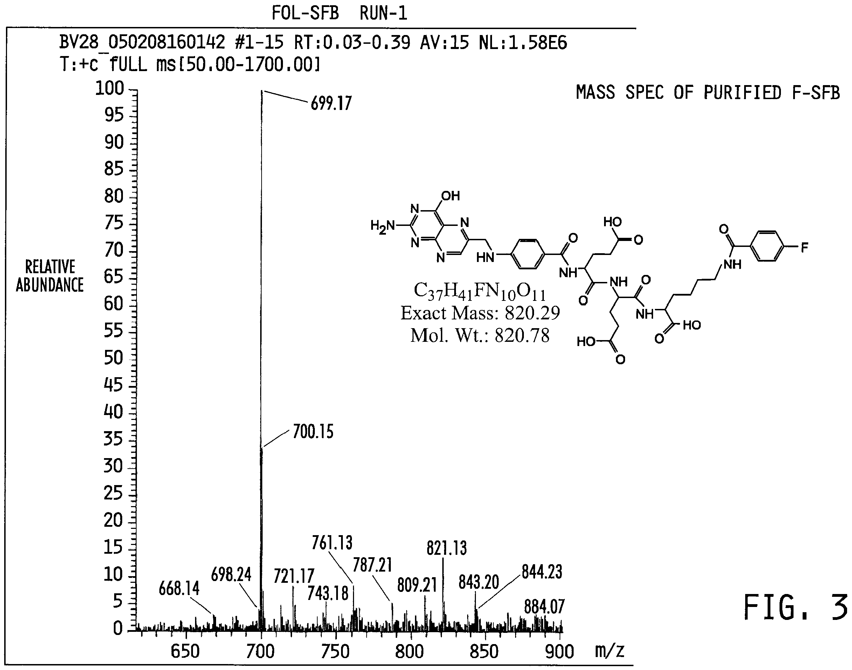

Crude and purified folate-SFB were analyzed by ESI-mass spectrometry using procedures known in the art. The results are shown in Figs. 2 and 3.

### EXAMPLE 6

### COMPETITIVE BINDING ASSAY

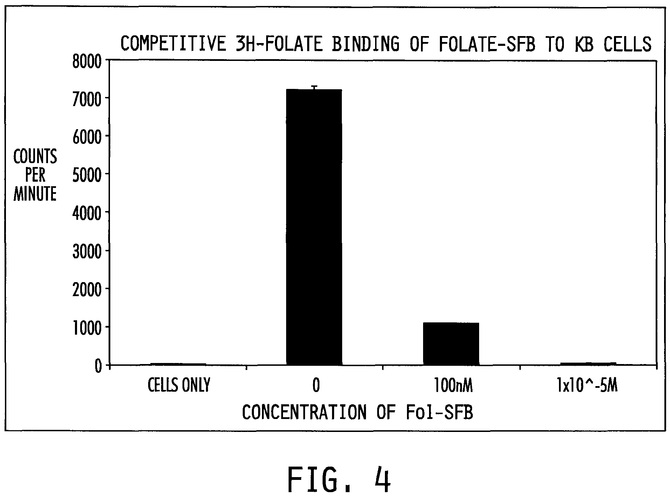

To determine whether folate-SFB binds to the folate receptor, a competitive binding assay was performed. KB cells were plated in 6-well plates using equal volumes of the cells in tissue culture medium. As shown in Fig. 4, the cells were incubated alone (first bar), with 100 nM ³H-folate (second bar), with 100 nM ³H-folate in the presence of 100 nM folate-SFB (third bar), or with 100 nM ³H-folate in the presence of 10 µM folate-SFB (fourth bar). The results show that folate-SFB competes with ³H-folate for binding to the folate receptor indicating that folate-SFB binds specifically to the folate receptor.

### EXAMPLE 7

### SYNTHESIS OF FOLATE-FBA

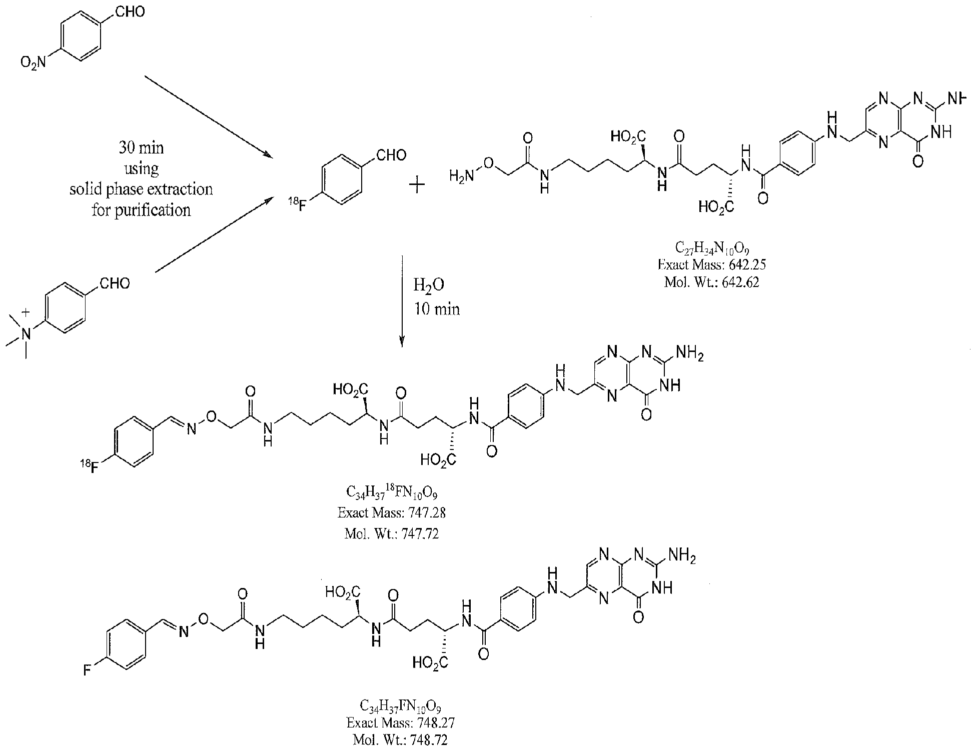

### EXAMPLE 8

### HPLC OF FOLATE-FBA

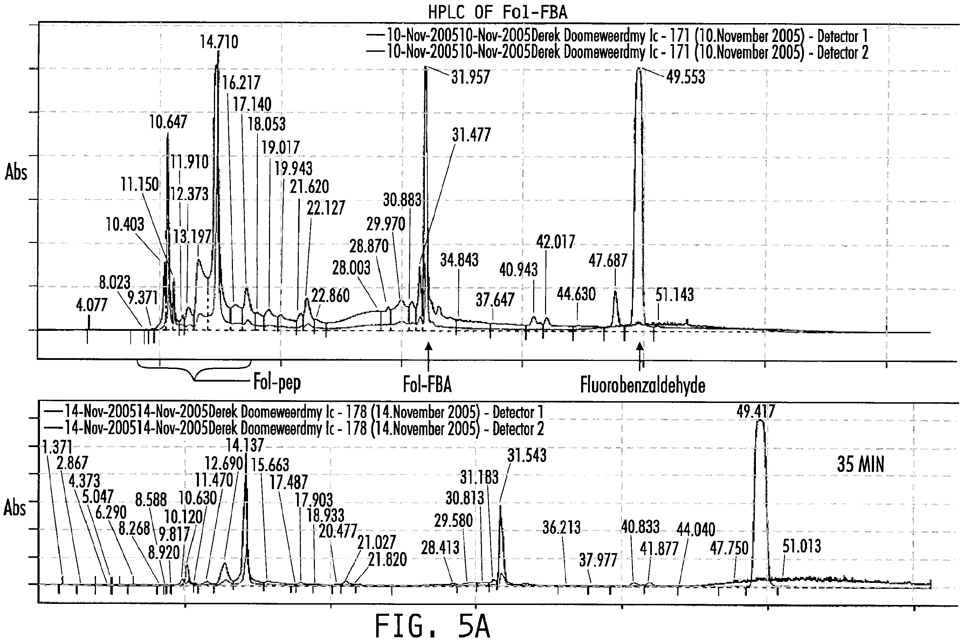

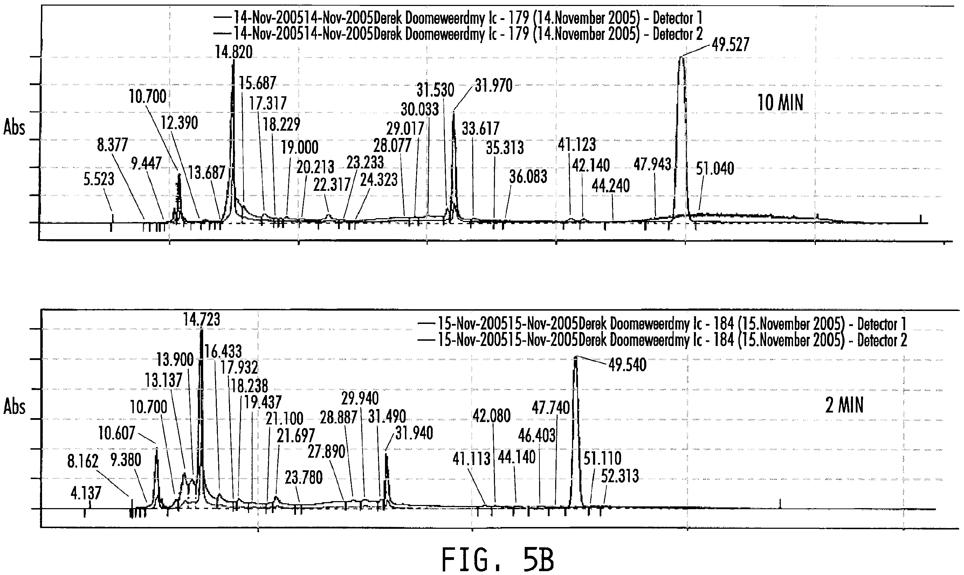

Folate-FBA was analyzed by high performance liquid chromatography (HPLC) using conditions similar to those described in Clinical Science, vol. 103: pp. 4S-8S (2002), but with the following modifications. The reverse phase HPLC was performed using a C 18 column and the conditions were water 0.1 % TFA in ACN at 77:23 for 10 min, 60:40 for 10 min, 50:50 for 10 min, 40:60 for 10 min, and using a flow rate of 1.0 ml/minute. The results are shown in Fig. 5 and indicate the separation of the folate-peptide, folate-FBA, and FBA alone on the column.

### EXAMPLE 9

### MASS SPECTRUM OF PURIFIED FOLATE-FBA

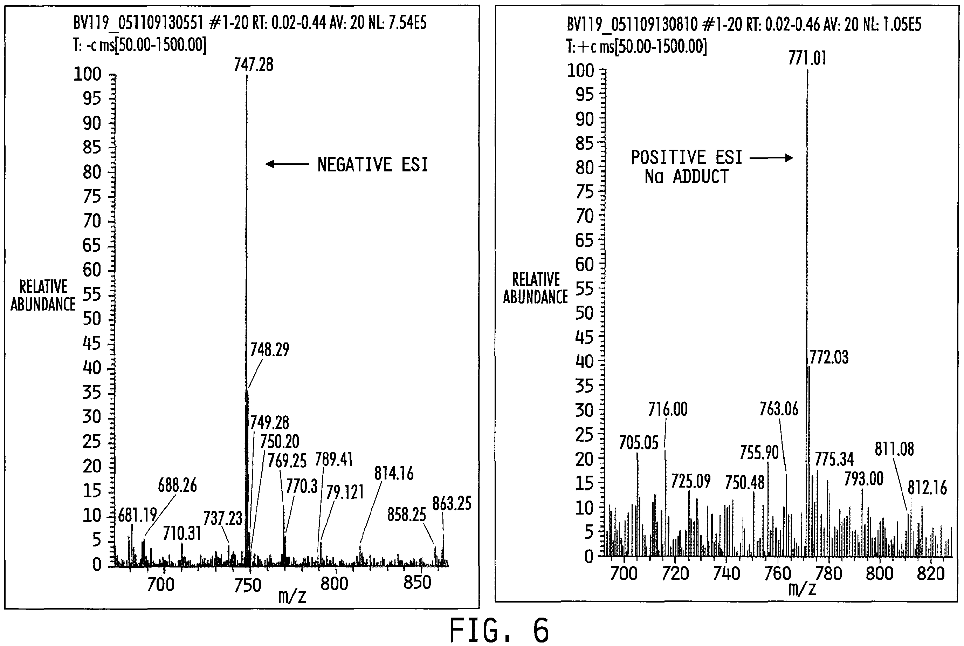

Folate-FBA and a sodium adduct of folate-FBA were analyzed by ESI-mass spectrometry using procedures known in the art. The results are shown in Fig. 6.

### EXAMPLE 10

### SYNTHESIS OF FOLATE CONJUGATE

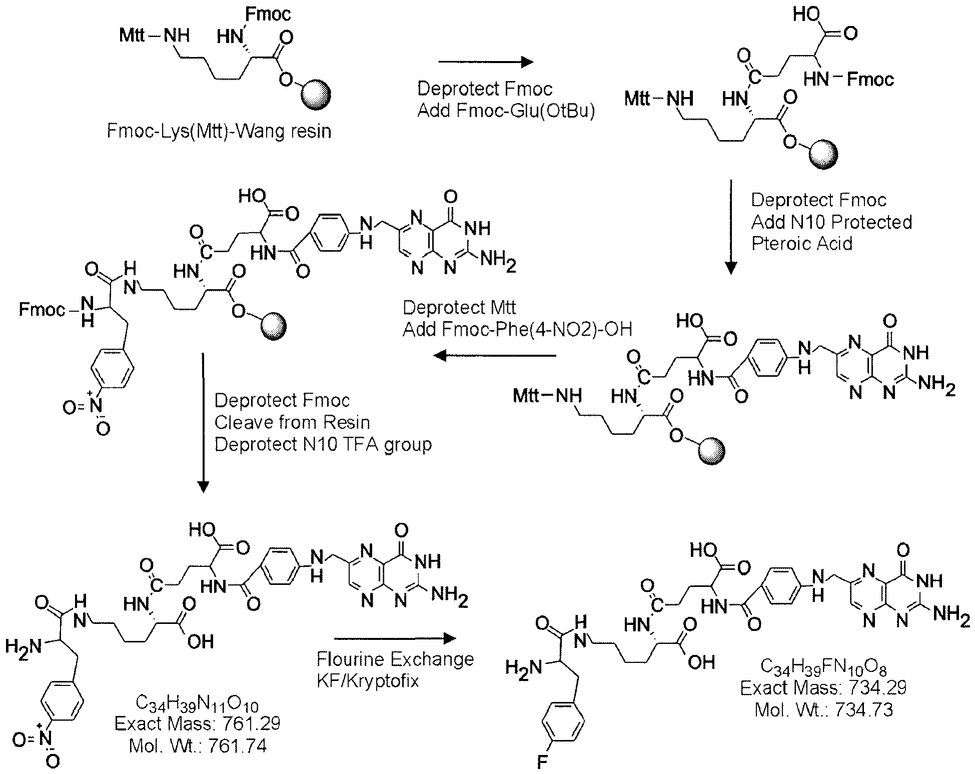

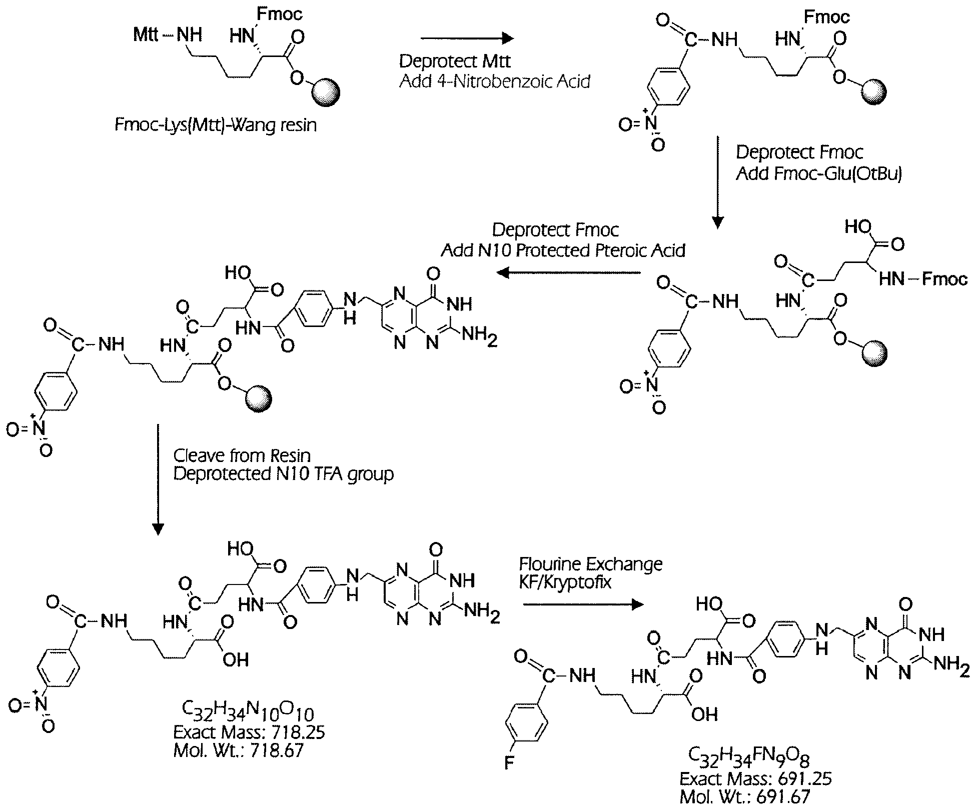

## Claims

1. A compound of the formula
L-X
wherein L is selected from folate, folinic acid, pteropolyglutamic acid, a tetrahydropterin, a dihydrofolate, a tetrahydrofolate, a 1-deaza folate, a 3-deaza folate, a 5-deaza folate, an 8-deaza folate, a 10-deaza folate, a 1,5 dideaza folate, a 5,10-dideaza folate, an 8,10-dideaza folate, a 5,8-dideaza folate, aminopterin, amethopterin, N¹⁰ -methylfolate, 2-deamino-hydroxyfolate, 1-deazamethopterin, 3-deazamethopterin, and 3'5'-dichloro-4-amino-4-deoxy-N¹⁰-methylpteroylglutamic acid,
wherein X comprises a radiophore comprising a radioisotope; wherein the radiophore comprises a group selected from a benzamidyl, a benzylic, a phenyl, a naphthyl, and a benzoxazolyl group; and
wherein the radioisotope is ¹⁸F.

2. The compound of claim 1 wherein L is conjugated to the radiophore through a linker.

3. The compound of claim 2 wherein the linker is selected from diamines, dextran, cellulose ethers, peptides, and polyethylene glycol.

4. The compound of claim 1 having the formula

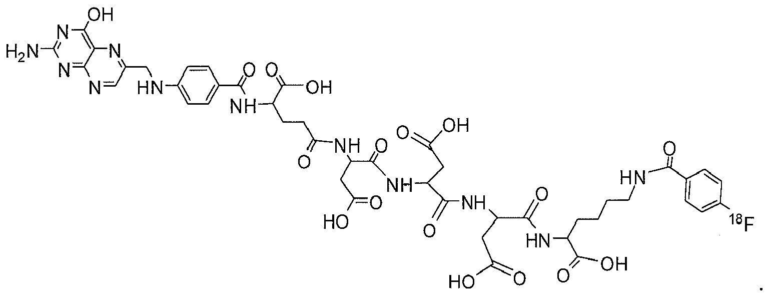

5. The compound of claim 1 having the formula

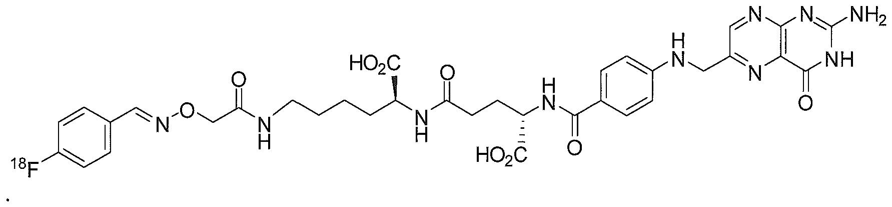

6. The compound of claim 1 wherein L is folate.

7. Use of a composition comprising a compound of the formula
L-X
wherein L is selected from folate, folinic acid, pteropolyglutamic acid, a tetrahydropterin, a dihydrofolate, a tetrahydrofolate, a 1-deaza folate, a 3-deaza folate, a 5-deaza folate, an 8-deaza folate, a 10-deaza folate, a 1,5 dideaza folate, a 5,10-dideaza folate, an 8,10-dideaza folate, a 5,8-dideaza folate, aminopterin, amethopterin, N¹⁰ -methylfolate, 2-deamino-hydroxyfolate, 1-deazamethopterin, 3-deazamethopterin, and 3'5'-dichloro-4-amino-4-deoxy-N¹⁰-methylpteroylglutamic acid;
wherein X comprises a radiophore comprising a radioisotope; wherein the radiophore comprises a group selected from a benzamidyl, a benzylic, a phenyl, a naphthyl, and a benzoxazolyl group; and
wherein the radioisotope is ¹⁸F,
for diagnosing a disease state selected from cancer, fibromyalgia,
rheumatoid arthritis, osteoarthritis, ulcerative colitis, Crohn's disease, psoriasis, osteomyelitis, multiple sclerosis, atherosclerosis, pulmonary fibrosis, sarcoidosis, systemic sclerosis, organ transplant rejection, lupus erythematosus, Sjögren's syndrome, glomerulonephritis, inflammatory lesions, infections of the skin, chronic inflammations, and inflammations due to injury.

8. The use of claim 7, wherein L in the compound is conjugated to the radiophore through a linker.

9. The use of claim 8 wherein the linker is selected from diamines, dextran, cellulose ethers, peptides, and polyethylene glycol.

10. The use of claim 7 wherein the compound has the formula

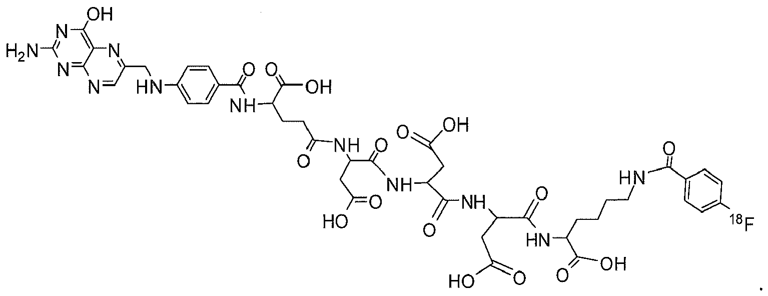

11. The use of claim 7 wherein the compound has the formula

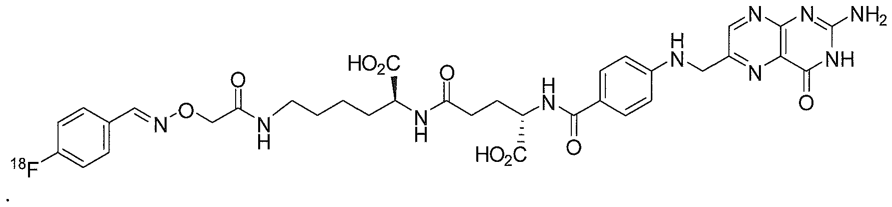

12. The use of claim 7 wherein the composition further comprises a pharmaceutically acceptable carrier.

13. The use of claim 12 wherein the pharmaceutically acceptable carrier is a liquid carrier selected from isotonic saline, 5% glucose, alcohols, glycols, esters, and amides.

14. The use of claim 7 wherein the composition is administered parenterally.

15. The use of claim 14 wherein the parenteral route of administration is selected from intravenous, intradermal, subcutaneous, intramuscular, and intraperitoneal.

16. The use of claim 7 wherein L is folate.
